# EUROPEAN PATENT APPLICATION

(11) **EP 1 038 529 A2**
(43) Date of publication of application: **27.09.2000**
(21) Application number: 00105361.0
(22) Date of filing: 17.03.2000
(51) Int. Cl.: A61K 35/36, A61P 31/18

(54) **Agent for increasing chemokine production**

(30) Priority: 19.03.1999 JP 7512299
(71) Applicant: NIPPON ZOKI PHARMACEUTICAL CO., LTD., Chuo-ku, Osaka (JP)
(72) Inventor: Konishi, Jin-emon, Nippon Zoki Pharmac. Co., Ltd., Osaka (JP); Ansari, Aftab A., Atlanta, GA 30322 (US); Gershwin, Eric M., c/o University of California, Davis, CA 95616 (US)
(74) Representative: HOFFMANN - EITLE

(57) **Abstract**

The present invention concerns the use of an extract from inflammatory tissue inoculated with vaccinia virus as active component in an agent for increasing chemokine production which is useful for the treatment of AIDS. The extracts from inflammatory tissue inoculated with vaccinia virus have increasing effects in the production of chemokines such as MIP-1α, MIP-1β and RANTES which are HIV-suppressive factors released from CD8⁺T cells. The extracts also have suppressive effects on SIV infection and on SIV replication. Therefore, such an extract is useful in an anti-HIV drug with new mechanisms of action which are different from those of known therapeutic agents for AIDS such as reverse transcriptase inhibitors and protease inhibitors, and an agent comprising this extract is very useful as a new drug for the treatment of AIDS with high safety.

## Description

### [Technical Field]

The present invention relates to a novel pharmacological action of an extract from inflammatory tissue inoculated with vaccinia virus. Particularly, it relates to an agent for increasing chemokine production containing an extract from inflammatory tissue inoculated with vaccinia virus as an effective component.

### [Prior Art]

Cocchi et al. purified and analyzed a substance having anti-HIV activity in the culture supernatant of CD8⁺T cells, and they found that β-chemokines (CC chemokines) such as MIP-1α (macrophage inflammatory protein 1α), MIP-1β and RANTES (regulated on activation, normal T expressed and secreted) are main components among the anti-HIV substances [Science, 270, p.1811 (1995)]. The anti-HIV activity in the culture supernatant of CD8⁺T cells was neutralized by an antibody against β-chemokines. In addition, the virus replications of human immunodeficiency virus (HIV) -1, HIV-2 and simian immunodeficiency virus (SIV) are suppressed by each of recombinant proteins of MIP-1α, MIP-1β and RANTES dose-dependently.

Most of therapeutic agents developed and used for AIDS are reverse transcriptase inhibitors such as azidothymidine and protease inhibitors, which are the drugs inhibiting the growth of virus per se. A cocktail therapy where two or more of such inhibitors are combined has been used in actual medical field and given some good effects on AIDS treatment. While the medical treatment by combination of two or more drugs such as cocktail therapy has been done as an effective therapy against AIDS, there is still a demand for further novel pharmaceutical agents for treatment of AIDS which have different mechanisms of action from currently used drugs. As mentioned above, MIP-1α, MIP-1β and RANTES are likely to be potential HIV-suppressive factors. It is therefore expected that a substance having an effect of increasing chemokine productions will be a novel anti-HIV agent based on the new mechanisms of action.

### [Problem]

The present inventors have conducted various tests and studies on pharmacological activities of an extract from inflammatory tissue inoculated with vaccinia virus. They recently have found that said extract has an increasing effect in the production of HIV-suppressive factors such as MIP-1α, MIP-1β and RANTES.

### [Solution]

An object of the present invention is to offer a pharmaceutical agent which is useful for the treatment of AIDS with the new mechanisms of action and high safety. Namely, the agent of the present invention scarcely shows side effects which are observed in the administration of agents presently used for AIDS treatment such as reverse transcriptase inhibitors and protease inhibitors.

### [Detailed Description]

It has been known that, living body produces various biofunction-regulating substances for maintaining its homeostasis against invasion of viruses, bacteria, etc. and against progress of diseases. These endogenous substances may regulate and normalize the biofunctions bilateral ways, i.e. a suppressing action to excessive reactions and an enhancing action to depression of functions. For example, there have been various reports on biofunction-regulating substances which are produced in inflammatory tissue inoculated with vaccinia virus, concerning the methods for extracting them from tissue and pharmacological activities thereof (refer, for example, to the Japanese Examined Patent Publications Sho-63/039,572 B, Sho-63/025,600 B, Hei-03/043,279 B and Japanese Patent 2,594,222).

There is an example of a commercially available drug preparation consisting of such biofunction-regulating substances extracted from inflammatory rabbit skin inoculated with vaccinia virus. This preparation is a drug containing non-proteinous active substances extracted and isolated from inflammatory rabbit skin inoculated with vaccinia virus as mentioned in pages 1,927 and 1,928 of "Drugs in Japan, Ethical Drugs" (22nd edition, 1998-1999; edited by Japan Pharmaceutical Information Center; published by Yakugyo Jiho Co., Ltd.). It is used for various diseases including low back pain, neck-shoulder-arm syndrome, periarthritis scapulohumeralis, arthrosis deformans, symptomatic neuralgia, post-herpetic neuralgia, pruritus due to dermatological diseases (such as eczema, dermatitis and urticaria), allergic rhinitis and sequelae of subacute myelo-optico-neuropathy (such as coldness, pain and dysesthesia). It is available as an ethical drug in the forms of injections (subcutaneous, intramuscular and intravenous) and of tablets.

The effective component of the agent for increasing chemokine production of the present invention is an extract containing non-proteinous biofunction-regulating substances produced in inflammatory tissue inoculated with vaccinia virus. A drug preparation of the extract from inflammatory rabbit skin inoculated with vaccinia virus which is listed in the above-mentioned "Drugs in Japan, Ethical Drugs" has been approved as an ethical drug, put into the market and available in Japan (trade name: Neurotropin). There are various kinds of extracts from inflammatory tissue inoculated with vaccinia virus. For example, the above-mentioned patent publications disclosed such various extracts which can be utilized as the effective component of the present invention. The manufacturing methods of the extracts and preferred doses thereof are also illustrated in the patent publications.

For example, the extract from inflammatory tissue inoculated with vaccinia virus is protein free and is positive for ninhydrin reaction (amino acid), orcinol reaction (orcinol-iron (III) chloride-hydrochloric acid method, pentose) and molybdenum blue reaction (phosphorus), and the aqueous solution thereof has an ultraviolet adsorption max of 265 to 275 and a pH of 6.0 to 8.3.

With regard to the routes of administration to the patient, subcutaneous, intramuscular and intravenous administrations by injections and oral administration by tablets are approved for the ethical drug preparation mentioned above. It is also possible to administer with a different dosage form which is most preferable for the therapy depending upon the type of the disease. The dose is to be suitably decided depending upon the kind of the extract from inflammatory tissue inoculated with vaccinia virus. For example, the dose of the ethical drug preparation approved in Japan is, in principle, 16 NU (Neurotropin Units) per day by oral administration and 3.6-7.2 Nu per day by injection, respectively (refer to the above "Drugs in Japan, Ethical Drugs", page 1,927) . The dose, however, may be appropriately increased or decreased depending upon the type of the disease, degree of seriousness, individual difference in the patients, method of administration, period of administration, etc.

The results of pharmacological tests concerning the novel actions of the extracts from inflammatory tissue inoculated with vaccinia virus are given below.

### [Examples]

### (1) Increasing effect in chemokine production

The peripheral blood mononuclear cells (PBMC) were prepared from the uninfected macaques with normal health according to a conventional method. Namely, heparinized blood was centrifuged at 150 × g for 10 minutes. The plasma was removed and then the cells were resuspended in a volume of RPMI 1640 media supplemented with 100 U/mL of penicillin, 100 µg/mL of streptomycin and 2 mM L-glutamine (heretofore referred to as media) equivalent to the volume of plasma removed. The blood suspension was then layered over Ficol-hypaque (Sigma Biochem. St. Louis, MO) and the gradient centrifugation was done at 450 × g. The mononuclear cells at the interface were aspirated and resuspended in media containing 10% of fetal calf serum. An aliquot of the cell suspension was counted, and the PBMC concentration was adjusted to the value required for the appropriate experiment. PBMC prepared as above were cultured in vitro at 2 × 10⁶ cells/mL with an optimum concentration of PHA-P in media containing varying concentration of the test drug (a drug preparation of an extract from inflammatory skin inoculated with vaccinia virus; trade name: Neurotropin). The cultures were incubated for 3 days at 37°C in a 5% CO₂ atmosphere. After washing, the cultures were infected with SIVmac251 for 2 hours in media containing the same concentration of the test drug. The cultures were washed to remove any free virus and then incubated continuously in media containing the same concentration of the test drug. The culture media containing the homologous concentration of the test drug was changed twice a week. After incubating the cultures for 10 days, each level of MIP-1α, MIP-1β or RANTES in the culture supernatant was measured by using enzyme immunoassay (EIA). An example of the results was shown in Table 1. The % values in Table 1 were calculated against the values of chemokines obtained in the absence of the test drug as 100%.

**Table 1**

| Chemokines | Addition of 0.1 µg/mL of the test drug | Addition of 1 µg/mL of the test drug |
|---|---|---|
| MIP-1α | 118.6% | 174.8% |
| MIP-1β | 131.4% | 212.6% |
| RANTES | 101.4% | 127.2% |

### (2) Suppressive effects on the ability of SIV to infect target cells and on SIV replication in target cells

As shown in Table 1, it was observed that the extract of the present invention exhibits increasing effect in the productions of MIP-1α, MIP-1β and RANTES which are recognized as HIV-suppressive factors. The present inventors further investigated suppressive effects of the extract from inflammatory tissue inoculated with vaccinia virus on the ability of SIV to infect target cells and on SIV replication in target cells. The procedures of culturing PBMC and SIV infections are carried out in the same manner as mentioned above. The viral load was quantitatively determined by measuring p27 level in the culture supernatant using p27 EIA kit (Coulter Corp. Hialeah, FL).

As a result, it was observed that the level of p27 in the fluid of the culture was significantly decreased. For example, p27 level in the supernatant of the culture which was incubated with the above-mentioned test drug (1 µg/mL) for 10 days after SIV infection was deceased to from 1/10 to 1/4 of that in the control. The p27 polypeptide is one of SIV-gag protein. The gag polypeptide of HIV corresponding to p27 is p24.

In addition to the EIA measurement of p27 level in the supernatant fluid, RT-PCR analysis of the same fluids and QC-RT-PCR analysis of an aliquot of the cells were also conducted. The results of these analyses mirrored the findings of the p27 level assay. Thus, the virus suppressive effects of the extract of the present invention were also seen at the level of viral RNA in the supernatant fluid and at the cellular RNA level.

### [Merit of the Invention]

It is apparent from the results of the above-mentioned pharmacological tests that the extracts of the present invention have increasing effects in the production of chemokines such as MIP-1α, MIP-1β and RANTES which are HIV-suppressive factors released from CD8⁺T cells. In addition, suppressive effects of the extracts of the present invention on SIV infection and on SIV replication were also observed.

The therapeutic agents for AIDS used at present, such as reverse transcriptase inhibitors and protease inhibitors, exhibit strong side effects. Therefore, there is still a strong demand for new drugs for AIDS having less side effects. There is a drug preparation of the extract from inflammatory skin inoculated with vaccinia virus commercially available and used for a long period of time, and it has been well known that the drug shows extremely little side effects. As mentioned above, the extracts of the present invention including the above drug have increasing effects in the production of chemokines such as MIP-1α, MIP-1β and RANTES. They have different mechanisms of action from reverse transcriptase inhibitors and protease inhibitors. Therefore, the agent of the present invention containing the extract from inflammatory tissue inoculated with vaccinia virus as an effective component is very useful as an anti-HIV drug with the new mechanisms of action and high safety.

## Claims

1. Use of an extract from inflammatory tissue inoculated with vaccinia virus for the manufacture of a medicament effective in increasing chemokine production.

2. Use according to claim 1, wherein the chemokine is MIP-1α, MIP-1β or RANTES.

3. Use according to claim 1 or 2, wherein the inflammatory tissue is skin tissue.

4. Use according to claims 3, wherein the inflammatory tissue is rabbit skin tissue.

5. Use according to any of claims 1 to 4, wherein the medicament is present in a form for injection.

6. Use according to any of claims 1 to 4, wherein the medicament is present in a form for oral administration.

7. Use according to any of claims 1 to 6, wherein the extract is protein free and is positive for ninhydrin reaction, orcinol reaction and molybdenium blue reaction.

8. Use according to any of claims 1 to 7, wherein the aqueous solution of the extract has an UV-adsorption maximum of 265-275 nm.

9. Use according to any of claims 1 to 8, wherein the aqueous solution of the extract has a pH value of 6,0 to 8,3.
